# EUROPÄISCHE PATENTSCHRIFT

(11) **EP 3 509 496 B1**
(45) Veröffentlichungstag und Bekanntmachung des Hinweises auf die Patenterteilung: **31.07.2024**
(21) Anmeldenummer: 17776951.0
(22) Anmeldetag: 12.09.2017
(51) Int. Cl.: A61B 6/00, A61B 5/00, G03B 17/24, G03C 3/00, G06Q 20/32, G06K 19/06, H04N 1/00, G01T 1/20, G06F 16/955, G03B 42/04, A61B 6/46, A61B 6/51

(54) **SYSTEM UND VERFAHREN ZUR BEREITSTELLUNG VON AUFNAHMEPARAMETERN**
SYSTEM AND METHOD FOR PROVIDING IMAGING PARAMETERS
SYSTÈME ET PROCÉDÉ POUR L'OBTENTION DE PARAMÈTRES DE PRISE DE VUE

(30) Priorität: 12.09.2016 DE 102016117051
(43) Veröffentlichungstag der Anmeldung: 17.07.2019
(73) Patentinhaber: Dürr Dental SE, 74321 Bietigheim-Bissingen (DE)
(72) Erfinder: WEBER, Michael, 71576 Burgstetten (DE); PHILIPPS, Bernd, 74199 Untergruppenbach (DE)
(74) Vertreter: Frenkel, Matthias Alexander
(86) Internationale Anmeldenummer: PCT/EP2017/072840
(87) Internationale Veröffentlichungsnummer: WO 2018/046750

(56) Entgegenhaltungen:
- US-A- 5 376 806
- US-A- 5 596 202
- US-A1- 2009 212 107
- US-A1- 2012 019 369
- US-A1- 2012 181 437

## Beschreibung

Die Erfindung betrifft ein System mit einer Röntgenaufnahmevorrichtung zur Aufnahme eines Röntgenbildes auf einer Speicherfolie und einer Auslesevorrichtung für die Speicherfolie. Solche Systeme werden heutzutage in der Röntgentechnik, beispielsweise in der Dentalmedizin, zur Aufnahme von Röntgenbildern verwendet. Die Speicherfolie weist zur Speicherung des Röntgenbildes ein Phosphormaterial auf, das in einer transparenten Matrix eingebettet ist. Dadurch entstehen Speicherzentren, die durch einfallendes Röntgenlicht in angeregte metastabile Zustände gebracht werden können. Belichtet man eine solche Speicherfolie in einer Röntgenapparatur, beispielsweise zur Aufnahme eines Bissflügels eines Patienten, so enthält die Speicherfolie ein latentes Röntgenbild in Form angeregter und nicht angeregter Speicherzentren.

Zum Auslesen der Speicherfolie wird diese in einer Auslesevorrichtung, beispielsweise einer Scanvorrichtung, punktweise mit Ausleselicht abgetastet, wodurch die metastabilen Zustände der angeregten Speicherzentren in einen Zustand gebracht werden, der unter Abgabe von Fluoreszenzlicht relaxiert. Dieses Fluoreszenzlicht wird mithilfe einer Detektoreinheit erfasst, sodass mit einer entsprechenden Auswerteelektronik das Röntgenbild sichtbar wird.

In der Medizin ist eine eindeutige Nachverfolgung und Zuordnung der Speicherfolien extrem wichtig. Zu diesem Zweck werden hier - wie auch in vielen anderen Logistikbereichen - Identifizierungssysteme eingesetzt.

US 2012/0019369 A1 beschreibt ein medizinisches Bildgebungssystem, bei welchem eine Auslesevorrichtung für eine Röntgenspeicherfolie ein Mittel zum Lesen eines Tags, wie beispielsweise eines Barcodes oder eines 2D-Barcodes, aufweist.

In ähnlicher Weise beschreibt auch US 2009/0212107 A1 eine Scanvorrichtung zum digitalen Extrahieren von Bildinformationen einer Röntgenspeicherfolie, die einen Laserscanner zum Abtasten der Röntgenspeicherfolie und ein Standardidentifikator-Lesegerät zum Auslesen eines an der Röntgenspeicherfolie angebrachten Standardidentifikators aufweist.

Die Druckschrift US 5,596202 A beschreibt die Verwendung eines Barcodes auf einer Speicherfolie unter Verwendung von fluoreszierendem Material.

Es ist eine Aufgabe der Erfindung, ein System und ein Verfahren zur Bereitstellung von Informationen für eine Auslesevorrichtung anzugeben, das einfach zu handhaben, zuverlässig und kostengünstig ist.

Diese Aufgabe wird durch ein System gemäß dem unabhängigen Anspruch 1, durch eine Speicherfolie gemäß dem weiteren unabhängigen Anspruch sowie durch ein Verfahren gemäß dem unabhängigen Verfahrensanspruch gelöst. Weitere Ausgestaltungen der Erfindung sind in den abhängigen Ansprüchen angegeben.

Das erfindungsgemäße System weist eine Röntgenaufnahmevorrichtung zur Aufnahme eines Röntgenbildes auf einer Speicherfolie und eine Scanvorrichtung für die Speicherfolie auf. Erfindungsgemäß ist vorgesehen, dass die Speicherfolie als Datenträger eine optisch lesbare Kennzeichnung mit Bereichen mit erhöhter Reflektivität und Bereichen mit geringerer Reflektivität aufweist. Des Weiteren ist vorgesehen, dass die Röntgenaufnahmevorrichtung oder/und die Scanvorrichtung eine Datenvorrichtung zum Lesen von auf dem Datenträger gespeicherten Informationen umfasst, wobei die Datenvorrichtung dazu ausgelegt ist, ein auf Basis der unterschiedlichen Reflektivitäten der optisch lesbaren Kennzeichnung erzeugbares Streulicht mittels des Ausleselichtstrahls der Scanvorrichtung zu erfassen. Bei der optisch lesbaren Kennzeichnung kann es sich beispielsweise um einen Strichcode oder einen QR-Code handeln. Die zweifache Nutzung der Scanvorrichtung kann ein besonders kostengünstiges und gleichzeitig effizientes Auslesen der auf dem Datenträger gespeicherten Informationen ermöglichen. Die ohnehin vorhandene Scanvorrichtung kann von der Datenvorrichtung so angesteuert werden, dass auch ein Lesen einer optisch erfassbaren Kennzeichnung wie ein Strichcode, ein QR-Code oder ähnliches erfasst werden kann. Es ist somit mit dem erfindungsgemäßen System möglich, für eine Aufnahme eines Röntgenbilds mit der Speicherfolie entsprechende Informationen dem Datenträger zu entnehmen. Dabei kann es sich beispielsweise um Aufnahmeparameter handeln, die für die Aufnahme mit dem Röntgengerät zu verwenden sind. Des Weiteren kann mittels der auszulesenden Informationen die Verwendungsanzahl der Speicherfolie erfasst werden, um einen mutmaßlichen Verschleiß der Speicherfolie berechnen oder abschätzen zu können.

Bei einer Ausführungsform des Systems kann vorgesehen sein, dass die Informationen einen die Speicherfolie eindeutig identifizierenden Identifikationscode darstellen. Somit kann durch ein Auslesen der auf den Datenträger gespeicherten Informationen die Speicherfolie eindeutig identifiziert werden. Dies ermöglicht eine speicherfolienbezogene Verknüpfung anderweitig erfasster Daten wie beispielsweise die Generierung und/oder Erfassung von Verschleißdaten. Des Weiteren können mit der eindeutigen Identifizierung auch beispielsweise damit verknüpfte und anderweitig gespeicherte Daten der Speicherfolie oder dem darauf gespeichertem Röntgenbild verknüpft werden. Es können auch zusätzlich auf dem Datenträger gespeicherte Informationen wie beispielsweise Aufnahmeparameter, Verschleißdaten oder ähnliches mit der eindeutigen Identifizierung verknüpft werden.

Bei einer erfindungsgemäßen Weiterbildung der Erfindung ist vorgesehen, dass die Datenvorrichtung dazu eingerichtet ist, die gelesenen Informationen an die Scanvorrichtung zu übertragen. Es stehen somit der Scanvorrichtung für die Speicherfolie beispielsweise bereits vor dem Auslesevorgang die auf dem Datenträger befindlichen Informationen zur Verfügung und das Auslesen der Speicherfolie kann beispielsweise bereits auf die Aufnahmeparameter angepasst werden, die bei der Aufnahme des Röntgenbildes verwendet wurden. Die Aufnahmeparameter können beispielsweise als zentral hinterlegte Daten vorhanden sein und mittels der gelesenen Informationen identifizierbar sein.

Bei den Aufnahmeparametern kann es sich beispielsweise um eine Spannung, eine Stromstärke, eine Belichtungszeit, eine Dosis, ein Dosisflächenprodukt, einen Blendenwert, Daten zu einem Patienten oder/und Daten zu einem Auftrag handeln. Das Aufzeichnen der genannten Aufnahmeparameter stellt eine Verknüpfung zwischen den genannten Parametern und dem auf der Speicherfolie befindlichen Röntgenbild dar und erlaubt somit eine Nachverfolgung der Röntgenaufnahme.

Die erfindungsgemäße Speicherfolie weist eine lichtempfindliche Schicht, insbesondere für die Speicherung eines Röntgenbildes auf und ist dazu ausgelegt, in einer Lichtschutzhülle aufbewahrt zu werden. Erfindungsgemäß ist vorgesehen, dass die Speicherfolie als Datenträger eine optisch lesbare Kennzeichnung, insbesondere einen Strichcode oder einen QR-Code, mit Bereichen erhöhter Reflektivität und Bereichen mit geringerer Reflektivität aufweist. Diese optisch lesbare Kennzeichnung ist somit an einer Stelle angebracht, die im üblichen Umgang mit der Speicherfolie nicht lesbar ist, da die Speicherfolie normalerweise in einer Schutzhülle untergebracht ist. Allerdings bietet die Anbringung einer optisch lesbaren Kennzeichnung die Möglichkeit, diese beispielsweise beim Auslesen der Speicherfolie zu erfassen. Bei dem Auslesen der Speicherfolie wird die lichtempfindliche Schicht mittels eines Ausleselaserstrahls aktiviert. In diesem Zusammenhang befindet sich die Speicherfolie also außerhalb der Lichtschutzhülle und ist somit einem Erfassen der optisch lesbaren Kennzeichnung zugänglich. Erfindungsgemäß wird das Auslesen direkt mit dem Ausleselichtstrahl der Speicherfolie durchgeführt. Nicht erfindungsgemäß kann auch eine andere Lichtquelle, beispielsweise auch mit einer den Auslesevorgang nicht beeinflussenden Wellenlänge, eingesetzt werden. Bei der mittels der optisch lesbaren Kennzeichnung kann es sich beispielsweise um eine Identifikation der Speicherfolie handeln.

Das erfindungsgemäße Verfahren zur Bereitstellung von Informationen für eine Scanvorrichtung weist die folgenden Schritte auf: Es wird mittels einer Röntgenaufnahmevorrichtung ein Belichtungsvorgang einer Speicherfolie durchgeführt, sodass ein Röntgenbild auf der Speicherfolie entsteht. Es wird die Speicherfolie mittels der Scanvorrichtung ausgelesen. Es wird der Datenträger mittels der Scanvorrichtung ausgelesen. Dabei ist die genannte Reihenfolge nicht zwingend. So können beispielweise Aufnahmeparameter, die bei einer Röntgenaufnahme verwendet werden sollen, vor dem Belichten der Speicherfolie aus dem Datenträger ausgelesen werden. Weiter kann vor oder nach dem Belichten eine die Speicherfolie charakterisierende Kennzeichnung der Speicherfolie von dem Datenträger gelesen werden.

Des Weiteren kann vorgesehen sein, dass der Schritt des Auslesens der Speicherfolie das Ausleseergebnis des Datenträgers berücksichtigt. Werden bei dem Auslesen der Speicherfolie beispielsweise die Aufnahmeparameter in Betracht gezogen, mit denen das Röntgenbild auf die Speicherfolie belichtet wurde, können unter Umständen die Auslesebedingungen für die Speicherfolie optimiert werden.

Die Erfindung wird nun unter Bezugnahme auf die beigefügten Zeichnungen näher erläutert. Es zeigen:
- Figuren 1A, 1B: in schematischer Darstellung Teile eines erfindungsgemäßen Systems;
- Figuren 2A-D: in schematischer Darstellung verschiedene Ausführungsformen einer erfindungsgemäße Speicherfolie;
- Figur 3: in einer schematischen Darstellung eine Ausführungsform einer Auslesevorrichtung; und
- Figur 4: ein Ablaufdiagramm eines erfindungsgemäßen Verfahrens.

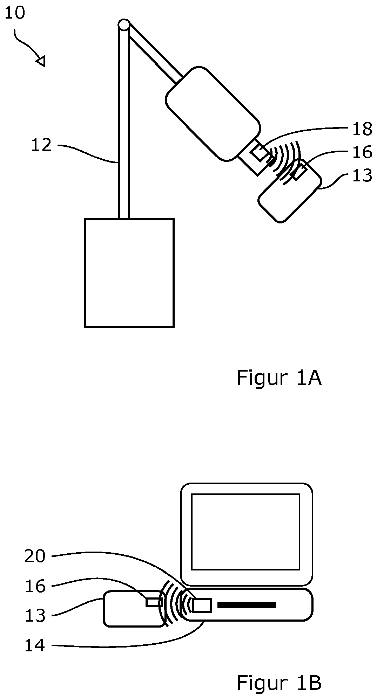

In den Figuren 1A und 1B ist ein System 10 zur Bereitstellung von Informationen dargestellt. Das System 10 umfasst eine Röntgenaufnahmevorrichtung 12 (Figur 1A) zur Belichtung einer Speicherfolie 13 mit einem Röntgenbild, wie sie beispielsweise in der Dentalmedizin eingesetzt werden, sowie eine Auslesevorrichtung 14 zum Auslesen des auf der Speicherfolie 13 befindlichen Röntgenbildes. Zur Aufnahme eines Röntgenbildes wird die Speicherfolie 13 üblicherweise in der Mundhöhle eines Patienten an geeigneter Stelle mittels nicht dargestellter Haltevorrichtungen angeordnet und über die Röntgenaufnahmevorrichtung 12 belichtet. Für die Belichtung sind an der Röntgenaufnahmevorrichtung 12 für die jeweilige Aufnahmesituation geeignete Aufnahmeparameter einzustellen. Diese Aufnahmeparameter umfassen beispielsweise eine Spannung, eine Stromstärke, eine Belichtungszeit, eine Dosis, ein Dosisflächenprodukt und/oder einen Blendenwert und bestimmen so die Aufnahmebedingungen. Unter den Aufnahmeparametern können sich aber auch patienten- oder auftragsspezifische Informationen befinden.

Die Speicherfolie 13 umfasst neben der eigentlichen röntgenstrahlenempfindlichen Struktur einen RFID-Transponder 16. Der RFID-Transponder 16 kann beispielsweise an oder in einer üblicherweise vorgesehenen lichtdichten Schutzhülle angeordnet sein. Der RFID-Transponder 16 arbeitet mit einer Schreib-/Lesevorrichtung 18 an der Röntgenaufnahmevorrichtung 12 und einem Lesegerät 20 an der Auslesevorrichtung 14 zusammen. Alternativ oder zusätzlich kann die Speicherfolie 13 eine optisch lesbare Struktur wie beispielsweise einen Strichcode aufweisen.

Die an der Röntgenaufnahmevorrichtung 12 vorgesehene Schreib-/Lesevorrichtung 18 ist dazu ausgelegt, einen Teil oder alle Aufnahmeparameter auf den RFID-Transponder 13 zu schreiben. Dazu können beispielsweise die vor dem Belichtungsvorgang eingestellten Sollwerte oder/und während oder nach dem Belichtungsvorgang erfasste Messwerte als Aufnahmeparameter erfasst und auf den RFID-Transponder 16 geschrieben werden. Zusätzlich kann die Schreib-/Lesevorrichtung 18 auch auf dem RFID-Transponder befindliche Informationen auslesen. Beispielsweise können bereits bei der Vorbereitung der Röntgenaufnahme auf der Speicherfolie 13 beispielsweise den Patienten, den Auftrag, das Röntgensystem oder/und das Gesamtsystem betreffende oder ähnliche Informationen auf dem RFID-Transponder 16 abgespeichert werden, die dann von der Röntgenaufnahmevorrichtung 12 ausgelesen und gegebenenfalls in der Konfigurierung des Belichtungsvorgangs der Speicherfolie 13 Eingang finden können.

Nach der erfolgten Belichtung muss das auf der Speicherfolie 13 befindliche Röntgenbild ausgelesen werden. Hierfür ist in der gezeigten Ausführungsform des Systems 10 die Auslesevorrichtung 14 vorgesehen. Bei der Auslesevorrichtung 14 kann es sich beispielsweise um eine Scanvorrichtung handeln, die mittels eines geführten Laserstrahls die metastabilen Zustände in der Speicherfolienmatrix aktiviert und so ein Auslesen des Röntgenbildes ermöglicht. Die in dem RFID-Transponder 16 enthaltenen Aufnahmeparameter können mittels des an der Auslesevorrichtung 14 vorgesehenen Lesegeräts 20 beispielsweise bereits vor dem Auslesevorgang der Speicherfolie 13 ausgelesen werden und gegebenenfalls für den Auslese-/Scanvorgang verwendet werden. Die Kenntnis der Aufnahmeparameter kann die Einstellung des Auslesevorgangs unter Umständen erleichtern.

Das bei der Auslesevorrichtung 14 vorgesehene Lesegerät 20 kann auch als Schreib-/Lesevorrichtung ähnlich der Schreib-/Lesevorrichtung 18 ausgeführt sein. So können nach dem Auslesen der Speicherfolie 13 noch auf dem RFID-Transponder 16 befindliche Informationen wieder gelöscht werden. Alternativ oder zusätzlich können ein Teil oder die gesamten Ausleseergebnisse wiederum auf den RFID-Transponder 16 geschrieben werden und so abgespeichert werden. Es kann auch ein Vermerk auf dem RFID-Transponder 16 abgelegt werden, der darauf hinweist, dass die Speicherfolie 13 bereits ausgelesen wurde.

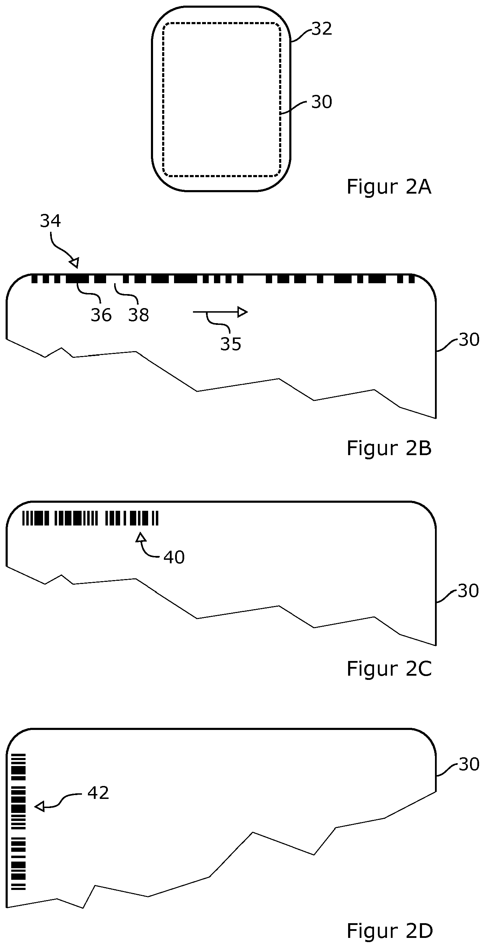

Die Figuren 2A-D zeigen eine Ausführungsform einer Speicherfolie 30. Die Speicherfolie 30 ist, wie in Figur 2A gezeigt, während ihrer Handhabung in einer Schutzhülle 32 eingeschoben. Die Schutzhülle 32 dient als mechanischer Schutz, um die empfindliche Speicherfolie 30 vor Verkratzen oder Knicken zu schützen. Gleichzeitig schützt die Schutzhülle 32 die Speicherfolie 30 vor unerwünschtem Lichteinfall, der das auf der Speicherfolie 30 befindliche latente Speicherbild zerstören oder eine noch unbelichtete Speicherfolie in unerwünschter Weise belichten würde. Für ein Auslesen ist die Speicherfolie 30 der Schutzhülle in geschützter Umgebung zu entnehmen und wird mit einem Ausleselicht punkt- oder zeilenweise abgetastet, wodurch die das Röntgenbild speichernden metastabilen Zustände der angeregten Speicherzentren relaxieren und Fluoreszenzlicht abgeben.

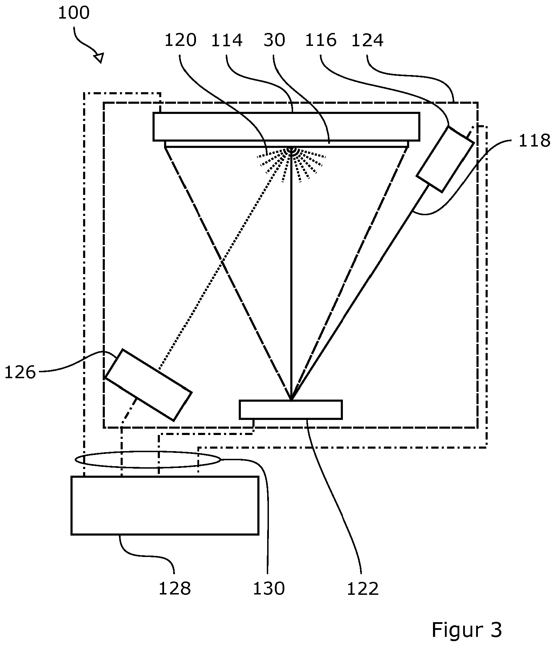

Figur 3 zeigt eine Scanvorrichtung 100 zum Auslesen einer solchen Speicherfolie 30, die in Form von durch Röntgenstrahlung angeregte metastabile Speicherzentren ein latentes Röntgenbild trägt.

Die Scanvorrichtung 100 weist eine Stützvorrichtung 114 für die Speicherfolie 30 auf. Beispielsweise kann die Speicherfolie 30 auf der Stützvorrichtung 114 mit Unterdruck so befestigt sein, dass die Speicherfolie 30, die im Allgemeinen flexibel ist, sich plan an die Stützfläche 114 anschmiegt.

Die Scanvorrichtung 100 umfasst ferner als Ausleselichtquelle einen Laser 116, der einen Ausleselichtstrahl 118 mit einer im Roten liegenden Wellenlänge erzeugt, mit dem die metastabilen Speicherzentren der Speicherfolie 30 zu Fluoreszenz angeregt werden können. Dieses Fluoreszenzlicht 120 liegt typischerweise im Blauen.

In der vorliegenden Ausführungsform der Scanvorrichtung 100 ist der Laser 116 so angeordnet, dass er den Ausleselichtstrahl 118 auf eine steuerbare Ablenkeinheit richtet. Die steuerbare Ablenkeinheit ist vorliegend als Spiegel 122 ausgebildet. Es sind aber auch andere Ablenkeinheiten außer Spiegel wie etwa Optiken oder dergleichen denkbar. Der Spiegel 122 kann als Mikrospiegel, insbesondere als MEMS-Komponente ausgeführt sein und so ein Abtasten der Fläche der Speicherfolie 30 ohne oder mit nur geringer Relativbewegung zwischen Spiegel 122 und Stützvorrichtung 114 ermöglichen. Alternativ kann der Spiegel 122 auch herkömmlich als rotierender Spiegel für einen Trommelscanner vorgesehen sein. In diesem Fall ist eine Relativbewegung zwischen der Stützvorrichtung 114 und dem Spiegel 122 mittels einer Transportvorrichtung (nicht abgebildet) realisiert.

Die Scanvorrichtung 100 kann ferner einen in der Zeichnung gestrichelt angedeuteten Reflektor 124 umfassen, der den gesamten Messraum um die Speicherfolie 30 herum lichtdicht umschließt, sodass das von der Speicherfolie 30 ausgehende Fluoreszenzlicht 120 zu einem Photodetektor 126 reflektiert wird. Um zu verhindern, dass gestreutes Ausleselicht 118 in den Photodetektor 126 gelangt, können geeignete Maßnahmen wie etwa ein dichroitisches Filtermaterial vorgesehen sein.

Zur Steuerung des Auslesevorgangs umfasst die Scanvorrichtung 100 eine Steuereinheit 128, die beispielsweise neben der Steuerfunktion auch Auswerte- oder Korrekturfunktionen wahrnehmen kann. Die Steuereinheit 128 selbst oder die Auswerte- oder/und Korrekturfunktonen können aber auch auf einem separaten Computer implementiert sein. Die Steuereinheit 128 ist mittels Leitungen 130 mit der Stützvorrichtung 114, dem Detektor 126, dem Laser 116 sowie dem Spiegel 122 verbunden.

Zum Auslesen steuert die Steuereinheit 128 den Laser 116 sowie den Spiegel 122 an und tastet die Speicherfolie 30 punktweise sequenziell mit dem Ausleselichtstrahl 118 ab. Dabei wird die Intensität des abgegebenen Fluoreszenzlichts 120 mithilfe des Photodetektors 126 erfasst und in der Steuereinheit 128 zur Ausgabe aufbereitet.

In den Figuren 2B-D sind drei verschiedenen Ausführungsformen einer Speicherfolie 30 dargestellt. Die in Figur 2B dargestellte Speicherfolie 30 weist an ihrer Oberkante eine Strichcode-Struktur 34 auf, die im Wesentlichen die komplette Breite der Speicherfolie 30 in Richtung 35 einer Abtastzeile überdeckt. Die Strichcode-Struktur 34 ist so ausgebildet, dass sie bei einem Auslesen der Speicherfolie wie beispielsweise mit der Scanvorrichtung 100 in Abtastrichtung des Ausleselichtstrahls 118 Bereiche 36 mit erhöhter Reflektivität für den Ausleselichtstrahl 118 und Bereiche 38 mit geringerer Reflektivität, beispielweise mit normaler Reflektivität, aufweist. Die Bereiche 36 mit erhöhter Reflektivität können beispielsweise als Streubereiche wirken. Bei einem Abtasten der Speicherfolie kann somit beispielsweise vor einem normalen Auslesevorgang das von der Strichcode-Struktur 34 bei einem zeilenweisen Abtasten entstehende Streulicht detektiert werden. Da es bei diesem Vorgang nicht auf eine hohe Ortsauflösung ankommt, kann das Streulicht beispielsweise mit einer einfachen Photodiode (nicht abgebildet) erfasst werden. Alternativ oder zusätzlich kann eventuell bei der geforderten geringen Empfindlichkeit auch der ohnehin vorhandene Photodetektor 1126 diese Aufgabe übernehmen.

Die Figuren 2C und D zeigen Abwandlungen. Im Unterschied zur Strichcode-Struktur der Figur 2B bedeckt die Strichcode-Struktur 40 nur einen Teil der Oberfläche der Speicherfolie 30 in Abtastrichtung 35. Bei der in Figur 2D gezeigten Abwandlung erstreckt sich eine Strichcode-Struktur 42 senkrecht zur zeilenweisen Abtastrichtung 35 und erfordert somit ein Detektieren des entstehenden Streulichts zu Anfang jeder Abtastzeile.

Die mittels des Auslesens bzw. Erfassens der Strichcode-Strukturen gewonnenen Informationen können beispielsweise zur Identifizierung von Daten wie beispielsweise Aufnahmeparametern dienen, die zentral abgelegt sind. Somit kann ebenfalls ein verbessertes Auslesen des eigentlichen Röntgenbildes mittels Informationen erreicht werden, die mittelbar über die Speicherfolie selbst erreichbar sind.

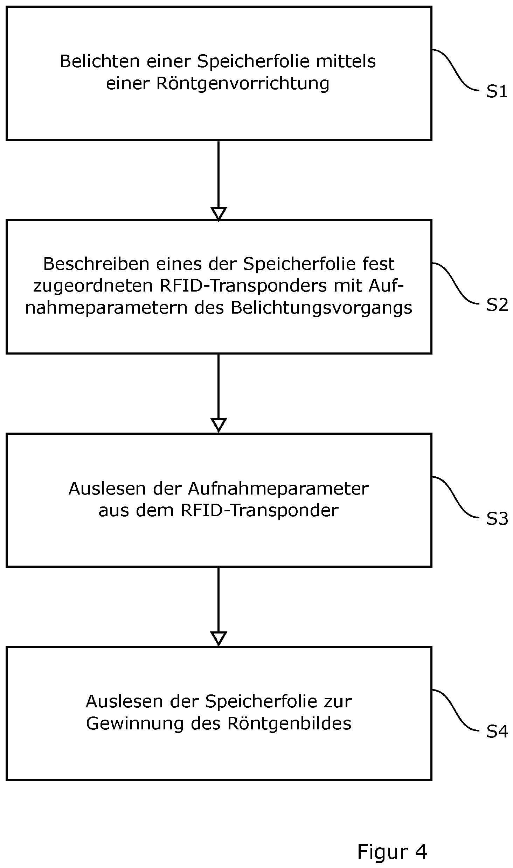

Figur 4 beschreibt eine Ausführungsform eines Verfahrens zur Bereitstellung von Informationen für eine Auslesevorrichtung. Das Verfahren weist folgende Schritte auf:
Eine Speicherfolie wird mittels einer Röntgenvorrichtung belichtet (S1). Bei dem Belichtungsvorgang wird ein Röntgenbild latent in der Speicherfolie erzeugt.

Ein der Speicherfolie fest zugeordneter RFID-Transponder wird mit Aufnahmeparametern des Belichtungsvorgangs beschrieben (S2). Der Vorgang des Beschreibens (S2) kann bereits vor dem Schritt des Belichtens (S1) stattfinden, wenn auf dem RFID-Transponder ausschließlich einzustellende Sollwerte zu hinterlegen sind. Alternativ oder zusätzlich kann der Vorgang des Beschreibens (S2) während oder nach dem Belichtungsvorgang (S1) stattfinden und es können alternativ oder zusätzlich auch während des Belichtungsvorgangs (S1) erfasste Messwerte auf den RFID-Transponder gespeichert werden. Für das Beschreiben des RFID-Transponders kann die Speicherfolie noch in der Röntgenvorrichtung verbleiben oder der Röntgenvorrichtung bereits entnommen sein.

Die auf dem RFID-Transponder befindlichen Aufnahmeparameter werden ausgelesen (S3). Die Speicherfolie kann nach dem Ende des Belichtungsvorgangs (S1) und dem Beschreiben des RFID-Transponders (S2) zu einer Auslesevorrichtung verbracht werden, um dort die Aufnahmeparameter auszulesen.

Das auf der Speicherfolie befindliche Röntgenbild wird mittels einer geeigneten Auslesevorrichtung ausgelesen (S4). Dabei kann es sich beispielsweise um eine Scanvorrichtung handeln, die mittels eines Lasers das latente Röntgenbild aktiviert und so ein Auslesen ermöglicht. Die Schritte des Auslesens der Aufnahmeparameter (S3) und des Auslesens der Speicherfolie (S4) können unabhängig voneinander erfolgen. Es kann aber vorgesehen sein, vor dem Auslesen der Speicherfolie (S4) die Aufnahmeparameter aus dem RFID-Transponder auszulesen (S3), um aus den Aufnahmeparametern Rückschlüsse auf geeignete Einstellungen für das Auslesen der Speicherfolie zu gewinnen.

## Patentansprüche

1. System (10) mit
- einer Röntgenaufnahmevorrichtung (12) zur Aufnahme eines Röntgenbildes auf einer Speicherfolie (13) und
- einer Scanvorrichtung (100) für die Speicherfolie (13), die als Ausleselichtquelle einen Laser (116) umfasst, mit dem für ein Auslesen der Speicherfolie (13) ein Ausleselichtstrahl (118) erzeugbar ist,
**dadurch gekennzeichnet, dass**
die Speicherfolie (13) als Datenträger eine optisch lesbare Kennzeichnung mit Bereichen (36) mit erhöhter Reflektivität und Bereichen (38) mit geringerer Reflektivität aufweist und
die Scanvorrichtung (100) eine Datenvorrichtung (18, 20) zum Lesen von auf dem Datenträger (16) gespeicherten Informationen umfasst, wobei die Datenvorrichtung (18, 20) dazu ausgelegt ist, ein auf Basis der unterschiedlichen Reflektivitäten der optisch lesbaren Kennzeichnung erzeugbares Streulicht mittels des Ausleselichtstrahls (118) der Scanvorrichtung (100) zu erzeugen und zu erfassen.

2. System nach Anspruch 1, wobei die Informationen einen die Speicherfolie (13) eindeutig identifizierenden Identifikationscode darstellen.

3. System nach einem der vorhergehenden Ansprüche, wobei die optisch lesbare Kennzeichnung ein Strichcode oder ein QR-Code sind.

4. Verfahren zur Bereitstellung von Informationen für eine Scanvorrichtung (100), mit den Schritten:
Durchführen (S1), mittels einer Röntgenaufnahmevorrichtung (12), eines Belichtungsvorgangs einer Speicherfolie (13);
Auslesen (S3) der Speicherfolie (13) mittels eines Ausleselichtstrahls (118) der Scanvorrichtung (100); und
Auslesen (S4) eines als optisch lesbare Kennzeichnung mit Bereichen mit erhöhter Reflektivität und Bereichen mit geringerer Reflektivität ausgeführten Datenträgers (16), indem ein auf Basis der unterschiedlichen Reflektivitäten der optisch lesbaren Kennzeichnung erzeugbares Streulicht mittels des Ausleselichtstrahls (118) der Scanvorrichtung (100) erzeugt und erfasst wird.

5. Verfahren nach Anspruch 4, wobei der Schritt des Auslesens (S3) der Speicherfolie (13) das Ausleseergebnis des Datenträgers (16) berücksichtigt.

## Claims

1. A system (10) with
- a radiographic device (12) for recording an X-ray image on a storage film (13) and
- a scanning device (100) for the storage film (13), which comprises, as a source of readout light, a laser (116) which generates a readout light beam (118) for readout of the storage film (13),
**characterised in that**
the storage film (13) includes, as a data carrier, an optically readable marking with regions (36) of increased reflectivity and regions (38) of lower reflectivity, and
the scanning device (100) comprises a data device (18, 20) for reading information stored on the data carrier (16), wherein the data device (18, 20) is configured to generate and detect a scattered light arising from the different reflectivities of the optically readable marking by means of the readout light beam (118) of the scanning device (100).

2. The system according to claim 1, wherein the information represents an identification code uniquely identifying the storage film (13).

3. The system according to one of the preceding claims, wherein the optically readable marking is a barcode or a QR code.

4. A method of providing information to a scanning device (100), comprising the steps of:
carrying out (51), by means of a radiographic device (12), a process of exposure of a storage film (13);
readout (S3) of the storage film (13) by means of a readout light beam (118) of the scanning device (100); and
readout (S4) of a data carrier (16) configured as an optically readable marking with regions of increased reflectivity and regions of lower reflectivity, in that a scattered light arising from the different reflectivities of the optically readable marking is generated and detected by means of the readout light beam (118) of the scanning device (100).

5. The method according to claim 4, wherein the step of reading out (S3) the storage film (13) takes into account the readout result of the data carrier (16).

## Revendications

1. Système (10) comprenant :
- un dispositif de radiographie (12) pour enregistrer une image radiographique sur une plaque mémoire (13) et
- un dispositif de balayage (100) pour la plaque mémoire (13), qui comprend comme source de lumière de lecture un laser (116) avec lequel un faisceau de lumière de lecture (118) peut être généré pour une lecture de la plaque mémoire (13),
**caractérisé en ce que**
la plaque mémoire (13) comprend, en tant que support de données, un marquage lisible optiquement avec des zones (36) à réflectivité élevée et des zones (38) à réflectivité plus faible, et
le dispositif de balayage (100) comprend un dispositif de données (18, 20) pour lire des informations stockées sur le support de données (16), le dispositif de données (18, 20) étant conçu pour générer et détecter une lumière diffusée pouvant être générée sur la base des différentes réflectivités du marquage lisible optiquement au moyen du faisceau de lumière de lecture (118) du dispositif de balayage (100).

2. Système selon la revendication 1, dans lequel les informations représentent un code d'identification identifiant de manière unique la plaque mémoire (13).

3. Système selon l'une des revendications précédentes, dans lequel le marquage lisible optiquement est un code à barres ou un code QR.

4. Procédé pour fournir des informations à un dispositif de balayage (100), comprenant les étapes de :
réalisation (S1), au moyen d'un dispositif de radiographie (12), d'un processus d'exposition d'une plaque mémoire (13) ;
lecture (S3) de la plaque mémoire (13) au moyen d'un faisceau lumineux de lecture (118) du dispositif de balayage (100) ; et
lecture (S4) d'un support de données (16) réalisé sous la forme d'un marquage lisible optiquement avec des zones à réflectivité élevée et des zones à réflectivité plus faible, en générant et en détectant une lumière diffusée pouvant être générée sur la base des différentes réflectivités du marquage lisible optiquement au moyen du faisceau lumineux de lecture (118) du dispositif de balayage (100).

5. Procédé selon la revendication 4, dans lequel l'étape de lecture (S3) de la plaque mémoire (13) tient compte du résultat de lecture du support de données (16).
